(19) Europäisches Patentamt
European Patent Office
Office européen des brevets

(11) EP 0 537 517 B1

(12) EUROPÄISCHE PATENTSCHRIFT

(45) Veröffentlichungstag und Bekanntmachung des Hinweises auf die Patenterteilung:
**22.01.1997 Patentblatt 1997/04**

(51) Int Cl.[6]: **C07D 317/46**

(21) Anmeldenummer: **92116316.8**

(22) Anmeldetag: **24.09.1992**

# (54) Verfahren zur Herstellung von 2,2-Difluorbenzo(1,3) dioxol-carbaldehyden und neuen Zwischenprodukten

Method for the preparation of 2,2-difluorobenzo(1,3)dioxolecarbaldehydes and intermediates

Procédé pour la préparation de 2,2-difluorobenzo(1,3)dioxolecarbaldéhydes et intermédiaires

(84) Benannte Vertragsstaaten:
**BE CH DE ES FR GB IT LI NL**

(30) Priorität: **07.10.1991 DE 4133155**

(43) Veröffentlichungstag der Anmeldung:
**21.04.1993 Patentblatt 1993/16**

(73) Patentinhaber: **BAYER AG**
**51368 Leverkusen (DE)**

(72) Erfinder:
- **Andres, Peter, Dr.**
**W-5653 Leichlingen 2 (DE)**
- **Marhold, Albrecht, Dr.**
**W-5090 Leverkusen 1 (DE)**

(56) Entgegenhaltungen:
**EP-A- 0 291 799** **DE-A- 4 029 444**

- **A.H. BLATT: 'Organic Syntheses, Collective Volume 2' 1946 , JOHN WILEY & SONS , NEW YORK, US, Seiten 549-550**
- **JOURNAL OF ORGANIC CHEMISTRY, Bd. 37, Nr. 4, 25. Februar 1972, WASHINGTON, DC, US, Seite 673 E.L. STOGRYN 'A new synthesis of 3,4-(difluoromethylenedioxy)benzaldehyde'**
- **AUSTRALIAN JOURNAL OF CHEMISTRY, Bd. 22, Nr. 7, 1969, EAST MELBOURNE, AU, Seiten 1563 - 1568 I.R.C. BICK, ET AL.: 'Synthesis of 3,4-dihydroxy-2- methoxybenzaldehyde: the use of methylenedioxy as a protecting group'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein neues Verfahren zur Herstellung von 2,2-Difluorbenzo(1,3)dioxol-carbaldehyden, die als wertvolle Ausgangsverbindungen für die Synthese hochaktiver Verbindungen zur Bekämpfung z.B. von Schädlingen im Pflanzenschutz gebraucht werden, und neue Zwischenprodukte.

Es ist bekannt, daß 2,2-Difluorbenzo(1,3)dioxol-carbaldehyde erhältlich sind, wenn man von 4-Methyl-benzo-(1,3) dioxol ausgeht, dieses zum 2,2-Dichlorprodukt mit Phosphorpentachlorid und Chlor umsetzt, das dann mit Flußsäure zur 2,2-Difluorverbindung umgesetzt wird. Dann wird die Methylgruppe bromiert und diese Gruppierung schließlich in bekannter Weise mit Urotropin in die Aldehydgruppe umgewandelt und somit wird der gewünschte 2,2-Difluor-benzo (1,3)dioxol-4-carbaldehyd erhalten (vgl. EP-A 291.799 = DE-A-3.716 652).

Nachteile dieses bekannten Verfahrens sind die Vierstufenreaktion. Ferner die schlechte Zugänglichkeit und Verfügbarkeit der jeweiligen Ausgangsmaterialien bei einer mittelmäßigen Gesamtausbeute des Verfahrens.

Weiterhin ist eine Synthese von 2,2-Difluor-benzo(1,3)-dioxol-5-carbaldehyd bekannt, die von 5-Brom-benzo[1,3] dioxol ausgeht, das chloriert wird zur 2,2-Dichlorverbindung, anschließend umgesetzt wird mit Fluorwasserstoffsäure in Gegenwart von Antimontrifluorid zur 2,2-Difluorverbindung und dann die daraus mit Butyllithium gewonnene Lithiumverbindung mit Dimethylformamid zu dem gewünschten Aldehyd umsetzt (vgl. E.L. Stogryn: J. Org. Chem. Vol. 37 (1972) Nr. 4, 673).

Diese Synthese hat technisch keine Bedeutung erlangt. Nachteile sind der Einsatz von Antimontrifluorid und Butyllithium, die erstens teuer sind und zweitens Probleme bei der Entsorgung, d.h. die Abfall- und Nebenprodukte davon, machen. Auch muß beim Transport und der Handhabung des Butyllithiums ein erhöhter Sicherheitsaufwand beachtet werden.

Der letzte Schritt Umsetzung des 2,2-Difluorbenzo[1,3]-dioxols mit z.B. Butyllithium oder generell mit Alkalimetall oder einer Alkali- oder Erdalkalimetallverbindung mit starker Anionbase wird u.a. beschrieben in EP-A-333.658. Die Nachteile sind identisch mit den oben

angegebenen und die verwendeten Ausgangsmaterialien können z.B, aus 1,3-Benzodioxol gewonnen werden, aus dem zuerst die 2,2-Dichlorverbindung hergestellt wird (vgl. DE-A-3 821 130) und daraus die Difluorverbindung (vgl. EP-A-41 131), die dann wie beschrieben formyliert wird.

Die Nachteile sind die bereits aufgezeigten.

Schließlich ist aus Org. Synth. Coll. Vol. 2, 549-550 (1946) und auch aus Austr. J. Chem. 22, 1563 - 1568 (1969) das 5-Dichlormethyl-2,2-dichlorbenzodioxol der Formel

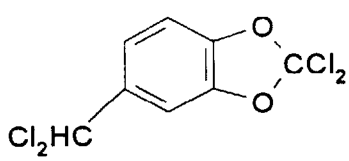

bekannt. Die entsprechende ortho-substituierte Verbindung, nämlich das 4-Dichlormethyl-2,2-dichlorbenzodioxol, wurde aber bisher noch nicht beschrieben.

Es wurde nun gefunden, daß man die 2,2-Difluor-benzo-[1,3]dioxol-carbaldehyde der allgemeinen Formel (I)

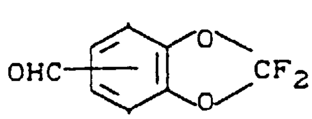

(I)

erhält, indem man Benz[1,3]dioxolcarbaldehyde der allgemeinen Formel (II)

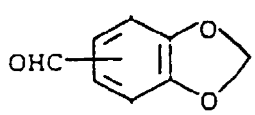

(II)

in erster Stufe gegebenenfalls in Gegenwart von Lösungs- oder Verdünnungsmitteln chloriert und die so erhältlichen chlorsubstituierten Benzo[1,3]-dioxole der allgemeinen Formel (III)

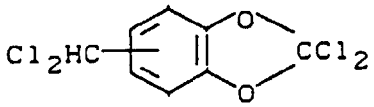

(III)

in einer zweiten Stufe mit Fluorwasserstoff partiell fluoriert und die so erhältlichen Verbindungen der Formel (IV)

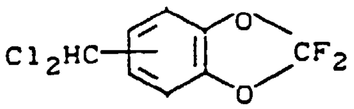

(IV)

in an sich bekannter Weise in einer 3. Stufe mit Urotropin umsetzt.

Es ist als ausgesprochen überraschend zu bezeichnen, daß gemäß der erfindungsgemäßen Umsetzung die gewünschten Produkte in hoher Reinheit und guten Ausbeuten erhalten werden. Vor allem ist es überraschend, daß in der zweiten Stufe die partielle Fluorierung der Tetrachlorverbindung zur 2,2-Difluorverbindung so glatt abläuft ohne irgendwelche nennenswerten Nebenreaktionen. Das erfindungsgemäße Verfahren besitzt gegenüber dem vorbekannten Verfahren eine Reihe von Vorteilen:

Erstens wird bei vergleichbaren, aber nicht identischen Ausgangsverbindungen ein eine Reaktionsstufe eingespart. Zweitens sind die verwendeten Benzo[1,3]dioxol-carbaldehyde auch in technischen Mengen gut zugänglich, d. h. bessere Zugänglichkeit und Verfügbarkeit der Startmaterialien, und außerdem liefert die Synthese die gewünschten Produkte in hoher Reinheit und guten Ausbeuten.

Verwendet man beispielsweise in 1. Stufe Benzo[1,3]-dioxol-4-carbaldehyd und Phosphorpentachlorid, in 2. Stufe Flußsäure und in bekannter Weise Urotropin in 3. Stufe, so läßt sich der Reaktionsablauf des erfindungsgemäßen Verfahrens durch das folgende Formelschema darstellen:

1.

2.

3.

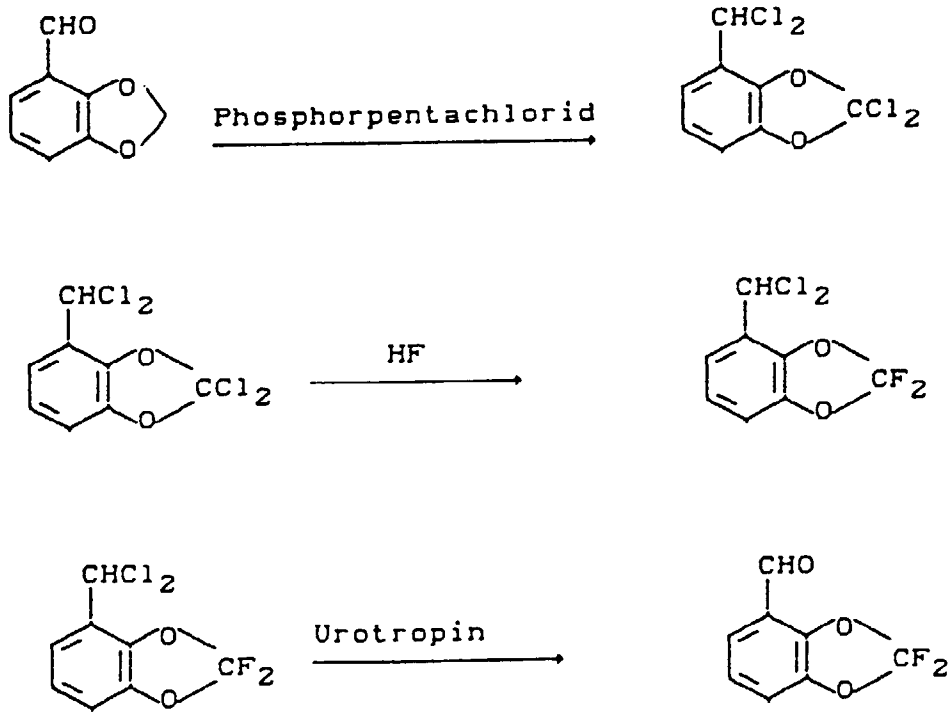


Die zur Durchführung des erfindungsgemäßen Verfahrens als Ausgangsstoffe benötigten Benzo[1,3]-dioxol-carbaldehyde der Formel (II) sind bekannt (vgl. Tetrahedronietters Nr. 38, 3361-3364 (1976)) und nach Analogieverfahren herstellbar.

Die weiterhin zu verwendenden Chlorierungsmittel in der 1. Stufe , der Fluorwasserstoff in der 2. Stufe und das Urotropin in der 3. Stufe sind gängige Chemikalien und käuflich zu erwerben.

Das als Zwischenprodukt auftretende 4-Dichlormethyl-2,2-dichlor-benzo(1,3)-dioxo und die 2,2-Difluor-dichlormethyl-benzo(1,3)-dioxole der Formel (IV) sind neu und ebenfalls Teil der Erfindung.

In der Stufe 1 werden Chlorierungsmittel benötigt. Es können alle üblichen Chlorierungsmittel verwendet werden wie z.B. Phosphorchloride, wie Phosphorpentachlorid, oder Phosphorchloride und Chlor, wie z.B. Phosphortrichlorid oder Phosphorpentachlorid und Chlor.

Bei Verwendung von Chlor werden Radikalstarter zugesetzt wie z.B. AIBN Azo-bis-isobutyronitril in äquivalenten Mengen von 0,001-0,05. Es kann auch in Gegenwart von energiereichem Licht wie UV-Licht gearbeitet werden. In der 1. Stufe wird bevorzugt ohne Lösungsmittel gearbeitet. Es kommen aber auch chlorierte Kohlenwasserstoffe in Frage, wie z.B, perchlorierte Kohlenwasserstoffe wie Tetrachlorkohlenstoff.

Die Temperaturen können in einem größeren Bereich variiert werden. Im allgemeinen arbeitet man bei Temperaturen zwischen 0°C und 220°C, vorzugsweise bei Temperaturen zwischen 80°C und 180°C, besonders bevorzugt bei Temperaturen zwischen 100°C und 160°C.

Das Verhältnis von eingesetztem Chlorierungsmittel, wie Phosphorpentachlorid zu Ausgangsmaterial der Formel (II) ist vorzugsweise 2:1 bis 4:1, bevorzugt 2,1:1.

In Stufe 2 kann gegebenenfalls in Gegenwart eines Lösungs- oder Verdünnungsmittels gearbeitet werden. Als solche kommen in Frage alkylierte Aromaten wie Toluol, chlorierte Aromaten wie Chlorbenzol. Bevorzugt wird ohne Lösungsmittel gearbeitet.

In der Stufe 2 liegt das Verhältnis Fluorwasserstoff zum Ausgangsprodukt der Formel (III) zwischen 2:1 und 20:1, bevorzugt zwischen 5:1 und 15:1 und besonders bevorzugt zwischen 7:1 und 10:1.

In Stufe 2 kann die Reaktionstemperatur in einem größeren Bereich variiert werden. Im allgemeinen arbeitet man bei Temperaturen zwischen -30°C und +80°C, bevorzugt zwischen -20°C und +40°C und besonders bevorzugt zwischen -10°C und +10°C.

Die Reihenfolge der Zugabe ist nicht von besonderer Bedeutung. In Abhängigkeit von der Reaktionstemperatur ist jedoch die Zugabe von Fluorwasserstoff zu den Verbindungen der Formel III bei Temperaturen > 0°C bevorzugt.

In der Stufe 3 wird in wäßriger Lösung gearbeitet.

In Stufe 3 kann die Reaktionstemperatur in einem größeren Bereich variiert werden. Im allgemeinen arbeitet man zwischen 20°C und 150°C, bevorzugt zwischen 60°C und 120°C und besonders bevorzugt zwischen 80°C und 110°C.

In der Stufe 3 beträgt das Verhältnis Urotropin zum Ausgangsmaterial der Formel (IV) zwischen 2:1 und 1:1, bevorzugt 1:1.

Bei der 1. Stufe wird bis zur Beendigung der Chlorwasserstoffentwicklung gewartet, dann falls verwendet das Lösungsmittel abdestilliert und bei Verwendung von Phosphorpentachlorid werden das Phosphoroxichlorid und Phosphor(III)chlorid abdestilliert und das Produkt im Vakuum destilliert.

Auch bei der 2. Stufe wird die Beendigung der Chlorwasserstoffentwicklung abgewartet, was z.B, bei -10°C ungefähr 3 Stunden dauert. Der überschüssige Fluorwasserstoff wird abdestilliert oder als eigene Phase abgetrennt. Reste von Fluorwasserstoff können beispielsweise mit CaO, NaF oder KF neutralisiert oder mit Wasser ausgewaschen werden.

Die 3. Stufe wird ungefähr nach 16 Stunden wäßrig extrahiert und anschließend destilliert.

Die mit Hilfe des erfindungsgemäßen Verfahrens erhältlichen 2,2-Difluorbenzo(1,3)dioxol-carbaldehyde können eingesetzt werden als Ausgangsverbindungen für hochaktive Arzneimittel, z. B. zur Bekämpfung von Kreislauferkrankungen (Vergleiche DE-A-3 716 652).

Die 2,2-Difluorbenzo(1,3)dioxol-carbaldehyde können natürlich auch als Ausgangsverbindungen für andere hochaktive Verbindungen im Pharmabereich und z. B. im Pflanzenschutzbereich verwendet werden.

<u>Herstellungsbeispiele</u>

<u>Beispiel 1</u>

Stufe 1.1

4-Dichlormethyl-2,2-dichlorbenzo[1,3]dioxol

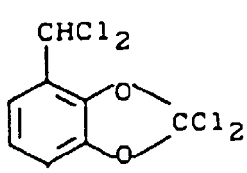

Unter Stickstoff gibt man zu 625,0 g (3,0 mol) Phosphorpentachlorid portionsweise 150,0 g (1,0 mol) 2,3-Methylendioxybenzaldehyd. Es wird 5 h (bis zum Ende der HCl-Entwicklung) auf 140°C erhitzt und gerührt. Dann destilliert man entstandenes Phosphoroxichlorid und Phosphor(III)chlorid ab und destilliert das Produkt im Vakuum. Man isoliert 260,0 g (95 % der Theorie) 4-Dichlormethyl-2,2-dichlorbenzo[1,3]dioxol mit einem Siedepunkt von 142°C/16 mbar.

Stufe 1.2

4-Dichlormethyl-2,2-difluorbenzo[1,3]dioxol

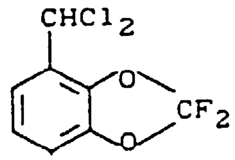

Zu 110,0 g (0,4 mol) 4-Dichlormethyl-2,2-dichlorbenzo[1,3]dioxol dosiert man bei -10°C 80,0 g (4,0 mol) wasserfreien Fluorwasserstoff. Anschließend wird bei -10°C noch 2 h kräftig gerührt. Die Hauptmenge an Fluorwasserstoff wird im Vakuum bis 50 mbar bei -10°C abgezogen. Nach Phasentrennung wird die organische Phase mit Eiswasser geschüttelt, getrennt, getrocknet und destilliert. Man erhält 77 g (80 % der Theorie) 4-Dichlormethyl-2,2-difluor-benzo [1,3]dioxol mit einem Siedepunkt von 90-92°C/16 mbar.

Stufe 1.3

2,2-Difluorbenzo[1,3]dioxol-4-carbaldehyd

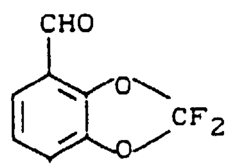

48,0 g (0,2 mol) 4-Dichlormethyl-2,2-difluorbenzo-[1,3]dioxol werden mit 28,0 g (0,2 mol) Urotropin (Hexamethylentetramin, Formin) in 200 ml 50 %iger Essigsäure 16 h unter Rückfluß gerührt. Dann setzt man 80 ml konz. Salzsäure zu und rührt noch 1 h unter Rückfluß. Nach dem Abkühlen wird auf 200 g Eis gegossen, dreimal mit je 300 ml Methyl-tert-butylether extrahiert, mit 300 ml Natriumhydrogencarbonat-Lösung und Wasser gewaschen, über Magnesiumsulfat getrocknet, eingeengt und destilliert. Man isoliert 32 g (86 % der Theorie) 2,2-Difluorbenzo[1,3]dioxol-4-carbaldehyd mit einem Siedepunkt von 105-106°C/50 mbar.

Beispiel 2

Stufe 2.1

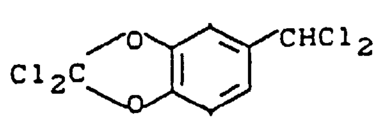

In einer Rührapparatur werden 700 g (3,36 Mol) Phosphorpentachlorid vorgelegt und 150 g (1,0 Mol) Methylendioxybenzaldehyd zugetropft. Dabei wird durch sehr langsames Bewegen des Rührers für Durchmischung gesorgt. Gegen Ende des Zutropfens wird die Reaktionsmischung normal rührbar und anschließend auf 120°C erhitzt. Die Chlorwasserstoffentwicklung dauert etwa 5 Stunden bei 120°C. Danach wird das entstandene Phosphoroxychlorid abdestilliert und anschließend das Produkt im Vakuum destilliert. Es werden 238 g (82,6 % der Theorie) 5-Dichlormethyl-2,2-dichlorbenzodioxol mit einem Siedepunkt 108-10°C/0,04 mbar erhalten.

Stufe 2.2

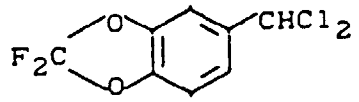

In einer Fluorierungsapparatur aus V4A-Stahl werden bei -15°C 50 ml Fluorwasserstoff vorgelegt und eine Mischung aus 130 g (0,45 mol) 5-Dichlormethyl-2,2-dichlor-benzodioxol und 100 ml trockenem Chlorbenzol unter Feuchtigkeitsausschluß zugetropft. Anschließend rührt man 4 h bei -10°C, erwärmt auf 0°C und rührt bis Ende der Chlorwasserstoffentwicklung nach (ca. 2 Stunden). Nach der Phasentrennung wird die organische Phase mit Eiswasser geschüttelt, getrennt, über Natriumsulfat getrocknet und destilliert.

Man erhält 79 g (73 % der Theorie) 5-Dichlormethyl-2,2-difluorbenzo-(1,3)-dioxol mit einem Siedepunkt 98-100°C/ 10 mbar.

Stufe 2.3

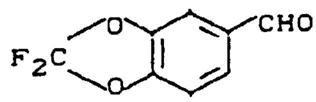

Eine Mischung aus 80 g (0,57 Mol) Urotropin, 150 ml Wasser und 80 g (0,33 Mol) 5-Dichlormethyl-2,2-difluorbenzo-(1,3)-dioxol wird für 2 Stunden bei 100°C gerührt, dann mit 100 ml Wasser und 50 ml konz. Salzsäure versetzt und nochmals 2 Stunden bei 100°C gerührt. Wasserdampfdestillation liefert nach Phasentrennung 58 g (94 % der Theorie) 2,2-Difluorbenzodixol-5-carbaldehyd (Reinheit ~ 99 %), mit einem Brechungsindex $n_D^{20}$: 1.4982.

<u>Vorprodukte</u>

<u>Beispiel a</u>:

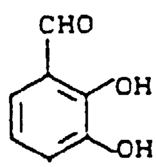

152,0 g (1,0 Mol) o-Vanillin und 200 ml azeotropische Bromwasserstoffsäure (47,5 %ig) werden in 500 ml Eisessig 2,5 h unter Rückfluß gerührt. Dann wird das Säuregemisch abdestilliert und der Rückstand einer fraktionierten Vakuumdestillation unterworfen. Die redestillierte Fraktion mit dem Siedepunkt 120-125°C/ 12 mbar wird durch Umkristallisation aus 1 1 Heptan (auch Hexan möglich, wird zurückgewonnen) gereinigt. Man erhält 97 g (70 % der Theorie) 2,3-Dihydroxybenzaldeyhd mit einem Schmelzpunkt von 105-107°C.

<u>Beispiel b</u>:

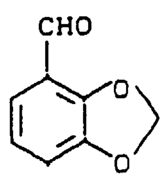

138,0 g (1,0 Mol) 2,3-Dihydroxybenzaldehyd, 415,0 g (3,0 Mol) Kaliumcarbonat, 4,7 g Kupferoxid, 255,0 g (3,0 mol) Methylenchlorid und 1500 ml destilliertes Dimethylformamid läßt man unter intensivem Rühren 7 h sieden. Das Reaktionsgemisch wird im Vakuum zur Trockene abgedampft und nach Zugabe von 1000 ml Eiswasser viermal mit je

300 ml Methyl-tert-butylether ausgeschüttelt, Die vereinigten Etherextrakte werden mit 5 %iger Natronlauge und Wasser gewaschen und über Natriumsulfat getrocknet. Danach wird der Methyl-tert-butylether abgezogen (kann wiederverwendet werden) und der Rückstand im Vakuum bei 92-93°C/1mbar destilliert. Man erhält 120 g (80 % der Theorie) 2,3-Methylendioxybenzaldehyd mit einem Schmelzpunkt von 32-33°C.

**Patentansprüche**

1. Verfahren zur Herstellung von 2,2-Difluorbenzo[1,3]dioxol-carbaldehyden der allgemeinen Formel (I)

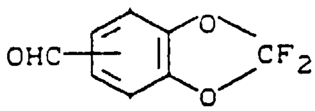

(I)

dadurch gekennzeichnet, daß man Benz[1,3]dioxalcarbaldehyde der allgemeinen Formel (II)

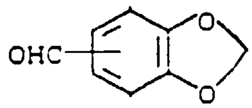

(II)

in erster Stufe gegebenenfalls in Gegenwart von Lösungs- oder Verdünnungsmitteln chloriert und die so erhältlichen chlorsubstituierten Benzo[1,3]-dioxole der allgemeinen Formel (III)

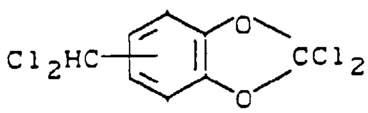

(III)

in einer zweiten Stufe mit Fluorwasserstoff partiell fluoriert und die so erhältlichen Verbindungen der Formel (IV)

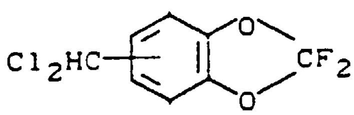

(IV)

in an sich bekannter Weise in einer 3. Stufe mit Urotropin umsetzt.

2. Verfahren nach Anspruch 1, dadurch gekennzeichnet, daß man in erster Stufe im Temperturbereich zwischen -0°C und 220°C, in zweiter Stufe im Temperaturbereich zwischen -30°C und +80°C und in dritter Stufe im Temperaturbereich zwischen 20°C und 150°C arbeitet.

3. Verfahren nach Anspruch 1, dadurch gekennzeichnet, daß man in erster Stufe im Temperaturbereich zwischen 80°C und 180°C, in zweiter Stufe im Temperaturbereich zwischen -20°C und 40°C und in dritter Stufe im Temperaturbereich zwischen 60°C und 120°C arbeitet.

4. Verfahren nach Anspruch 1, dadurch gekennzeichnet, daß in Stufe 1 zur Chlorierung Phosphorchloride, oder Phosphorchloride und elementares Chlor mit einem Radikalstarter oder energiereichem Licht verwendet werden.

5. 2,2-Dichlor-dichlormethyl-benzo[1,3]dioxol der Formel

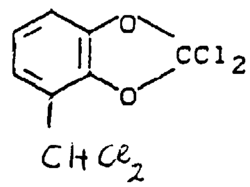

6. 2,2-Difluor-dichlormethyl-benzo[1,3]dioxole der allgemeinen Formel (IV)

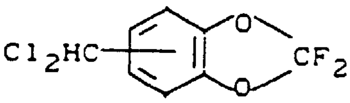

(IV)

**Claims**

1. Process for the preparation of 2,2-difluorobenzo-[1,3]-dioxole-carbaldehydes of the general formula (I)

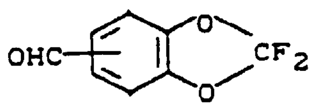

(I)

characterised in that benzo[1,3]dioxole-carbaldehydes of the general formula (II)

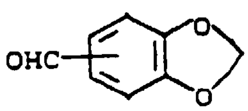

(II)

are chlorinated, if appropriate in the presence of solvents or diluents, in a first stage, the chlorine-substituted benzo [1,3]dioxoles of the general formula (III)

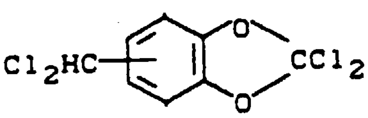

(III)

thus obtainable are partly fluorinated with hydrogen fluoride in a second stage, and the compounds of the formula (IV)

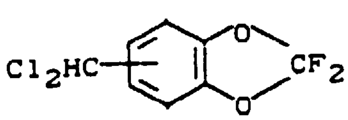

(IV)

thus obtainable are reacted with urotropin in a manner which is known per se in a 3rd stage.

2. Process according to Claim 1, characterised in that the first stage is carried out in the temperature range between -0°C and 220°C, the second stage is carried out in the temperature range between -30°C and +80°C and the third stage is carried out in the temperature range between 20°C and 150°C.

**3.** Process according to Claim 1, characterised in that the first stage is carried out in the temperature range between 80°C and 180°C, the second stage is carried out in the temperature range between -20°C and 40°C and the third stage is carried out in the temperature range between 60°C and 120°C.

**4.** Process according to Claim 1, characterised in that phosphorus chlorides, or phosphorus chlorides and elemental chlorine, with a free radical initiator or high-energy light, are used for the chlorination in the first stage.

**5.** 2,2-Dichloro-dichloromethyl-benzo[1,3]dioxoles of the formula

**6.** 2,2-Difluoro-dichloromethyl-benzo[1,3]dioxoles of the general formula (IV)

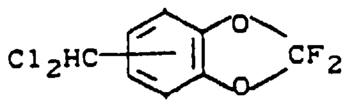

(IV)

**Revendications**

**1.** Procédé pour la préparation de 2,2-difluorobenzo[1,3]dioxol-carbaldéhydes répondant à la formule générale (I)

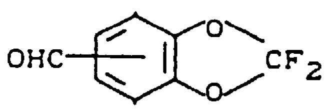

(I)

caractérisé en ce que, dans une première étape, éventuellement en présence de solvants ou de diluants, on soumet à une chloration des benz[1,3]dioxolcarbaldéhydes répondant à la formule générale (II)

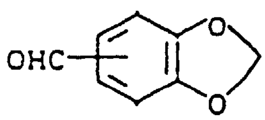

(II)

dans une deuxième étape, on soumet à une fluoration partielle avec de l'acide fluorhydrique les benzo[1,3]dioxols chlorosubstitués ainsi obtenus répondant à la formule générale (III)

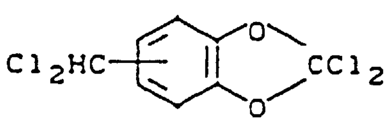

(III)

dans une troisième étape, d'une manière connue en soi, on fait réagir avec de l'urotropine les composés ainsi obtenus répondant à la formule (IV)

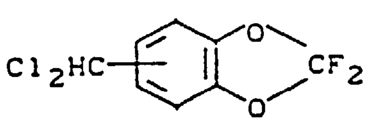

(IV)

**2.** Procédé selon la revendication 1, caractérisé en ce qu'on travaille, dans la première étape, dans le domaine de température entre -0°C et 220°C, dans la deuxième étape, dans le domaine de température entre -30°C et +80°C, et dans la troisième étape, dans le domaine de température entre 20°C et 150°C.

3. Procédé selon la revendication 1, caractérisé en ce qu'on travaille, dans la première étape, dans le domaine de température entre 80°C et 180°C, dans la deuxième étape, dans le domaine de température entre -20°C et 40°C, et dans la troisième étape, dans le domaine de température entre 60°C et 120°C.

4. Procédé selon la revendication 1, caractérisé en ce qu'on utilise, dans l'étape 1, à des fins de chloration, des chlorures de phosphore ou des chlorures de phosphore et du chlore élémentaire avec un agent d'amorçage radicalaire ou avec de la lumière riche en énergie.

5. 2,2-dichloro-dichlorométhyl-benzo[1,3]dioxol répondant à la formule

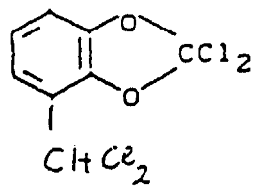

6. 2,2-difluoro-dichlorométhyl-benzo[1,3]dioxols répondant à la formule générale (IV)

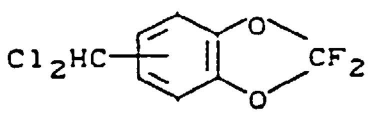

(IV)